# EUROPEAN PATENT APPLICATION

(11) **EP 4 806 345 A1**
(43) Date of publication of application: **16.09.2026**
(21) Application number: 25162950.7
(22) Date of filing: 11.03.2025
(51) Int. Cl.: A61B 5/08, A61M 16/00, A61B 5/085

(54) **COST-BASED EXCITATION DESIGN FOR RESPIRATORY MONITORING PURPOSES**

(71) Applicant: Vrije Universiteit Brussel, 1050 Ixelles (BE)
(72) Inventor: KEYMOLEN, Andy, 1090 Jette (BE); LATAIRE, John, 1160 Oudergem (BE); JONCKHEER, Joop, 2550 Kontich (BE); MARCHAL, Antoine, 1160 Auderghem (BE); VANDERSTEEN, Gerd, 1050 Ixelles (BE); VERBANCK, Sylvia, 1090 Jette (BE)
(74) Representative: Calysta NV

(57) **Abstract**

System for respiratory oscillometry for measuring a parameter of a respiratory system of a patient comprising: an excitation means configured to superimpose an oscillatory excitation signal on the ventilation of the patient; a ventilation sensor for measuring a ventilation excitation measurement; an excitation response processing means configured to determine based on the ventilation excitation measurement of the patient the parameter. An excitation signal generation means generates the oscillatory excitation signal, in particular a number, amplitude and/or phase of one or more excitation frequencies of the signal, based on a ventilation reference measurement of the patient. A computer program to estimate the parameters parametrically or non-parametrically.

## Description

### Technical Field

The present invention relates to system, method and computer program for respiratory oscillometry.

### Prior art

In clinical practice, respiratory mechanical parameters sensitive to the peripheral region can currently only be identified via invasive techniques when patient activity is present. The primary research focus has been to expand non-invasive methods, which have proven effective in controlled ventilation, to supportive ventilation modes. Current efforts primarily address the issue of patient effort through regularization techniques. However, the identifiability of these parameters depends on the validity of the applied prior knowledge, which often includes assumptions such as passive expiration, absence of ventilation asynchrony, no inspiratory coughs, or a specific shape/periodicity of the breathing effort is required. Patients recovering from respiratory failure frequently violate these assumptions, leading to inaccurate estimations. Additionally, even with precise modelling of the disturbances, existing methods rely on the ventilation waveform for excitation. In cases where clinicians reduce pressure support to zero-such as in constant positive airway pressure (CPAP) mode there is no driving pressure, leaving no excitation to identify the patient's respiratory mechanics.

Respiratory oscillometry (RO) offers an advantage by introducing an external excitation signal, which could theoretically overcome this identification challenge in CPAP mode. It measures via a mouthpiece the resistance and reactance of the airways using pressure oscillations, typically superimposed on normal breathing. However, breathing disturbances limit the use of low frequencies, which are essential for assessing the peripheral region and identifying the same parameters commonly used in clinical practice. Consequently, most low-frequency RO approaches are invasive. For outpatient measurements, i.e. measurements outside of a hospital, non-invasive low-frequency RO techniques have been developed. Although fixed frequency excitation signals have been sufficient for measurements above 5 Hz, they are inadequate at lower frequencies, where patient breathing introduces significant noise and compromises measurement quality. It was demonstrated that adapting the excitation signal based on individual patient data could improve the quality of measurements. An adaptive excitation signal design protocol was proposed, which selects optimal excitation frequencies for each patient by positioning them between ventilation harmonics. This approach reduces interference from patient breathing and improves measurement quality. This adaptive excitation design principle can enable low-frequency measurements during mechanical ventilation, also when patient activity is present, and allow estimation of clinically relevant mechanical parameters. While the method showed promise for respiratory monitoring during mechanical ventilation on ICU patients, challenges remained. Specifically, the method frequently failed during pressure support ventilation due to irregular breathing patterns or apneas, where ventilation harmonics either disappeared or were masked by noise. Additionally, even under ideal conditions, questions remain regarding the feasibility of exciting more frequencies (and thereby gathering more information) without compromising measurement quality.

In Keymolen et al., "Low-frequency respiratory oscillometric measurements during mechanical ventilation," in 2023 IEEE International Symposium on Medical Measurements and Applications (MeMeA), Jeju, Korea, Republic of: IEEE, Jun. 2023, pp. 1-6. doi: 10.1109/MeMeA57477.2023.10171930 the inventors suggested a method for using RO in pressure controlled mechanical ventilation to select the frequency values of the excitation frequencies of the oscillatory excitation signal by arranging them between the harmonics of the mechanical ventilation. However, this works only for mechanical ventilation which fully replaces the ventilation because already in assisted mechanical ventilation, there are no clear harmonics due to the noise of the respiration of the patient and other patient activity. This can also not be applied to spontaneously breathing patients.

B. Daróczy and Z. Hantos. Generation of optimum pseudorandom signals for respiratory impedance measurements. International journal of bio-medical computing, 25(1):21-31, 1990 discloses to design the amplitudes of the excitation frequencies based on an expected breathing noise. However, since the expected breathing noise is designed based on a model, very often the model assumptions do not apply for the real patients and thus the robustness of the method is not strong enough for a wide application of this method. Furthermore, the authors propose applying the optimized excitation signal across all patients.

WO 2016/186836 discloses a method for multi-frequency oscillatory ventilation in which the ventilation waveform is improved for gas delivery to a pulmonary region. While specifically improving the ventilation signal based on patient data, they aim to improve the ventilation functionality and not a measurement functionality.

### Brief summary of the invention

It is the object of the invention to improve respiratory oscillometry such that it can robustly measure a parameter of the respiratory system of the patient in many different situations, especially for measuring peripheral regions of the respiratory system.

The object is solved by a system for respiratory oscillometry according to the independent claim.

By determining the amplitudes, number of frequencies and/or phases of the excitation frequencies based on the ventilation reference measurement of the patient, thus based on a specific measurement of the ventilation of the patient, the oscillatory excitation signal can be optimized so that it can also be used in the very noisy frequency region below 5 Hz which is close to the frequencies of the ventilation of the patient. This allows to use the same respiratory oscillometry for different patients, for different types of mechanical ventilation and even for naturally/spontaneously breathing patients. The amplitude of each excitation frequency is made large enough to provide results with at least the minimal required quality. The invention solves all key challenges for respiratory mechanical measurements. RO is non-invasive, it provides accurate measurements even at low frequencies for the excitation frequencies (below 5 Hz) to measure peripheral regions, it is ventilation mode independent (and can even be used for spontaneously breathing patients) and it is finally robust versus patient activity.

The object is further solved by a system for respiratory oscillometry for measuring a respiratory parameter of a respiratory system of a patient comprising: a ventilation interface to connect with the respiratory system of the patient; an excitation means configured to superimpose an oscillatory excitation on a ventilation of the patient based on an oscillatory excitation signal; a ventilation sensor for measuring the ventilation of the patient, wherein the ventilation sensor is configured to perform a ventilation excitation measurement of the ventilation of the patient superimposed by the oscillatory excitation; an excitation response processing means configured to determine based on the ventilation excitation measurement of the patient a response of the respiratory system on the oscillatory excitation signal, and to determine based on the response of the respiratory system on the oscillatory excitation signal the respiratory parameter; an output means for outputting the determined respiratory parameter; an excitation signal generation means configured to generate the oscillatory excitation signal based on patient specific data.

The object is solved by a method for respiratory oscillometry for measuring a respiratory parameter of a respiratory system of a patient or for designing a patient specific oscillatory excitation signal for respiratory oscillometry of a patient, the method comprising the steps of: receiving patient specific data of the patient; generating an oscillatory excitation signal based on patient specific data; transmitting the oscillatory excitation signal generated to an excitation means for respiratory oscillometry for superimposing the generated oscillatory excitation signal on a ventilation of the patient. Preferably, the method comprises further the steps of: receiving a ventilation excitation measurement from a ventilation sensor, wherein the ventilation excitation measurement was performed during an oscillatory excitation of the ventilation of the patient with the generated oscillatory excitation signal; determining based on the ventilation excitation measurement of the patient a response of the respiratory system on the oscillatory excitation signal and/or to determine (based on the response of the respiratory system on the oscillatory excitation signal) the respiratory parameter; and giving out the determined respiratory parameter or response via an output means.

The object is further solved by a computer program comprising instructions configured to perform the steps of the above method when executed on a processor.

The object is solved by a method for respiratory oscillometry for measuring a respiratory parameter of a respiratory system of a patient, the method comprising the steps of: connecting a ventilation interface with the respiratory system of the patient; providing patient specific data of the patient; generating, in an excitation signal generation means, an oscillatory excitation signal based on the patient specific data; superimposing, via an excitation means, an oscillatory excitation on a ventilation of the patient via an oscillatory excitation signal; measuring, in a ventilation sensor, the ventilation of the patient during the superimposed oscillation excitation to obtain a ventilation excitation measurement of the ventilation of the patient; determining, in an excitation response processing means, based on the ventilation excitation measurement of the patient the respiratory parameter (preferably obtained based on a response of the respiratory system on the oscillatory excitation determined based on the ventilation excitation measurement); and outputting, via an output means, the determined respiratory parameter.

By determining the ventilation excitation signal based on patient specific data, the oscillatory excitation signal can be optimized so that it can also be used in the very noisy frequency region below 5 Hz which is close to the frequencies of the ventilation of the patient. This allows to use the same respiratory oscillometry for different patients, for different types of mechanical ventilation and even for natural breathing patients.

The object is solved by a mechanical ventilator comprising a system for respiratory oscillometry as described above. This system for respiratory oscillometry is particularly interesting for mechanical ventilation as it allows to control a ventilated patient non-invasively at frequency values close to the ventilation frequency below 5 Hz. Especially, when using a ventilation reference measurement measured during mechanical ventilation of the patient, the designed oscillatory excitation signal allows to avoid the noise generated by the mechanical ventilator. The present invention is particularly interesting for assisted mechanical ventilators which support also the natural breathing of the patient and have complex frequency spectra which make the respiratory oscillation work only with a patient specific design of the oscillation excitation signal.

The following embodiments show further features of the previously disclosed improvements.

In one embodiment, the oscillatory excitation signal comprises one or more excitation frequencies, wherein one or more signal parameters of the oscillatory excitation signal are determined based on a ventilation reference measurement of the patient, wherein the one or more signal parameters determined based ventilation reference measurement are one or more of the following: a number of the one or more excitation frequencies, one or more amplitude(s) of the one or more excitation frequencies and one or more phase(s) of the one or more excitation frequencies. Each of those signal parameters have the potential to increase significantly the quality of the design of the excitation signal, if the patient specific ventilation reference measurement is considered.

In one embodiment, the patient specific data of the patient contains a ventilation reference measurement of the patient, wherein the one or more signal parameters determined based on the patient specific data are based on the ventilation reference measurement.

In one embodiment, the ventilation reference measurement is a measurement of the ventilation of the patient without superimposed excitation oscillation.

In one embodiment, the ventilation reference measurement is a measurement of the ventilation of the patient with an oscillatory excitation. The ventilation reference measurement with the oscillatory excitation is obviously different from the ventilation excitation measurement for which the excitation signal is designed. Thus, for the ventilation reference measurement, an oscillatory excitation signal designed for a previous measurement is used.

In one embodiment, the ventilation reference measurement is measured by the ventilation sensor. This has the advantage that the ventilation reference measurement reflects also the noise of the ventilation sensor and/or of the system.

In one embodiment, the ventilation reference measurement is measured with a different ventilation sensor.

In one embodiment, a frequency spectrum of the ventilation reference measurement is computed, wherein the amplitude of each of the one or more excitation frequencies are determined based on the frequency spectrum. Preferably, the amplitude for each excitation frequency depends on the frequency spectrum at a frequency value of this excitation frequency and a predetermined additional amplitude value. This allows to optimize the amplitude of each excitation frequency based on the noise situation at or close to the frequency value of the excitation frequency and thus obtain a good signal to noise ratio for the ventilation excitation measurement for each excitation frequency without using too much amplitude.

In one embodiment, the additional amplitude value depends on a user input or depends on a measurement objective such as signal to noise ratio, parameter uncertainty, entropy, ... . The additional amplitude value can be selected by a user input or can be predefined.

In one embodiment, the one or more signal parameters are determined further based on a total amplitude budget. By distributing the total amplitude budget on the excitation frequencies allows to optimize the amplitude of each excitation frequency without exceeding a total amplitude budget which could harm or disturb the patient during the ventilation.

In one embodiment, the total amplitude budget depends on user input. This allows to change the amplitude budget, if a patient feels unwell or if the amplitude budget causes problems with the ventilation of the patient. For very sensitive patients with maybe weak lungs, the amplitude budget could be reduced.

In one embodiment, the number of the excitation frequencies are determined based on the determined amplitude of each excitation frequency and the total amplitude budget available. The amplitude budget concept is a powerful tool for selecting the number of excitation frequencies. For example, if a potential excitation frequency requires an amplitude which together with the remaining amplitudes of the other excitation frequencies goes over the total amplitude budget, the excitation frequency could be removed.

In one embodiment, the number of excitation frequencies, the frequency values of the excitation frequencies and the amplitudes of the excitation frequencies are selected by an optimization criterium distributing the total amplitude budget over the determined number of excitation frequencies. This allows to optimize the selection of the number of excitation frequencies, their frequency values and their amplitudes based on the patient specific ventilation reference measurement and the amplitude budget. The optimization criteria allow to select how this optimization can be done. Preferably, the optimization criterium is user selectable, e.g. can be signal to noise ratio, parameter uncertainty, entropy, ...

In one embodiment, the frequency values of the frequency spectrum are divided into a plurality of predefined frequency regions, wherein for each predefined frequency region at least one excitation frequency is selected based on a lowest necessary amplitude in this frequency region, if there is sufficient amplitude budget left to not exceed the total amplitude budget.

In one embodiment, the frequency values and their respective amplitudes of each of the one or more excitation frequencies are determined based on the frequency spectrum of the reference ventilation measurement.

In one embodiment, the excitation signal comprises excitation frequencies smaller than 5Hz, preferably lower than 4,5 Hz, preferably lower than 4Hz, preferably lower than 3Hz. The current RO system or method is particularly useful for measuring respiratory parameters with excitation frequencies close to the respiratory / ventilation frequencies.

In one embodiment, the one or more signal parameters of the oscillatory excitation signal are determined based on a previous respiratory oscillometry measurement of the patient. The one or more signal parameters of the oscillatory excitation signal are determined based on a previous ventilation excitation measurement, a previous oscillatory excitation signal and/or a previous respiratory parameter of the respiratory system of the patient of the previous respiratory oscillometry measurement of the patient. For example, the frequency value(s) of the excitation signal can be selected based on the frequency value(s) of the previous excitation signal. For example, if there was not sufficient amplitude to have an excitation frequency in a certain frequency region in the previous RO measurement, the oscillatory excitation signal could be designed such that in this excitation signal, a frequency value in the frequency region which was not represented in the previous RO measurement is selected.

In one embodiment, the number of excitation frequencies are selected based on the patient specific data, preferably based on the ventilation reference measurement.

In one embodiment, the phase of each excitation frequency is selected based on the patient specific data, preferably based on the ventilation reference measurement.

In one embodiment, the frequency value of each of the one or more excitation frequencies are selected based on the patient specific data, preferably based on the ventilation reference measurement.

In one embodiment, the frequency value and amplitude of each of the one or more excitation frequencies are selected based on the patient specific data, preferably based on the ventilation reference measurement. Especially, when combining the selection of the amplitude and the frequency value of each excitation frequency, the designed excitation signal improves significantly the oscillatory respiratory measurement, even in difficult frequencies below 5 Hz and even in noisy ventilation modes. Preferably, also the phase of each of the one or more excitation frequencies is selected based on the patient specific data, preferably based on the ventilation reference measurement. Adding also the phase to the list of signal parameters which are selected patient specifically increases further the robustness of the respiratory oscillometry measurement.

In one embodiment, the number of excitation frequencies, the frequency value and amplitude of each of the one or more excitation frequencies are selected based on the patient specific data, preferably based on the ventilation reference measurement. By selecting these three signal parameters, the excitation signal can be highly customized to the patient and provides subsequently robust and low-error measurements of the respiratory parameter of the patient. Preferably, they are determined further based on an available total amplitude budget. This concept allows to select the number of frequencies, their frequency values and amplitudes based on the available budget. Preferably, also the phase of each of the one or more excitation frequencies is selected based on the patient specific data, preferably based on the ventilation reference measurement.

In one embodiment, the oscillatory excitation signal comprises two or more excitation frequencies or is configured to contain two or more excitation frequencies.

In one embodiment, the one or more signal parameters of the oscillatory excitation signal are determined based on the frequency spectrum of the ventilation reference measurement of the patient.

In one embodiment, the excitation signal is not a therapeutic excitation signal, i.e. has not the function to improve the ventilation of the patient, but to improve the measurement of the excitation response in the ventilation measurement. In other words, the excitation signal is optimized to improve the measurement of the respiratory system.

In one embodiment, the respiratory parameter of the patient determined based on the ventilation excitation measurement is obtained by a parametric model, i.e. is obtained based on the combination of the frequency spectrum of the ventilation excitation measurement at multiple different frequencies corresponding to the excitation frequencies (in contrast to non-parametric parameters which are based on only one excitation frequency). This has several advantages for the present invention. First, the change of the frequency values of the excitation frequencies used for the excitation signal leads to the fact that computed non-parametric parameters of subsequent measurements are not comparable, because measured at different frequencies. Therefore, the use of parameters identified from parametric models makes them comparable also for subsequent measurements independent from the used frequency values for the excitation frequencies. In addition, parametric models require normally lower amplitudes which allows to use more excitation frequencies with the same amplitude budget (or to lower the amplitude budget).

In one embodiment, the ventilation reference measurement contains a pressure measurement of the ventilation.

In one embodiment, the ventilation reference measurement contains a flow measurement of the ventilation.

In one embodiment, the ventilation reference measurement contains a pressure measurement and a flow measurement of the ventilation.

In one embodiment, the mechanical ventilator is configured to perform an assisted mechanical ventilation, wherein the ventilation sensor is configured to measure the ventilation excitation measurement during the assisted mechanical ventilation. Examples for assisted ventilation is pressure-support ventilation or continuous positive airway pressure

Other embodiments according to the present invention are mentioned in the appended claims and the subsequent description of an embodiment of the invention.

### Brief description of the Drawings

Fig. 1 shows an example of the system for respiratory oscillometry.
Fig. 2 shows an example of the method for respiratory oscillometry.
Fig. 3 shows an example of the method for generating the excitation signal.
Fig. 4 shows a frequency spectrum of the ventilation reference measurement of a ventilation of a patient under volume-controlled ventilation.
Fig. 5 shows a frequency spectrum of the ventilation reference measurement of a ventilation of a patient under pressure-supported ventilation.
Fig. 6 shows a smoothed version of the frequency spectrum of Fig. 5 highlighting the local maxima and minima.
Fig. 7 shows a frequency spectrum of a ventilation reference measurement with low noise regions and the two types of amplitudes for potential excitation frequencies.
Fig. 8 shows a more detailed schematic for another example how to find the excitation frequencies and their amplitudes

In the drawings, the same reference numbers have been allocated to the same or analogue element.

### Detailed description of an embodiment of the invention

Other characteristics and advantages of the present invention will be derived from the following non-limitative description, and by making reference to the drawings and the examples.

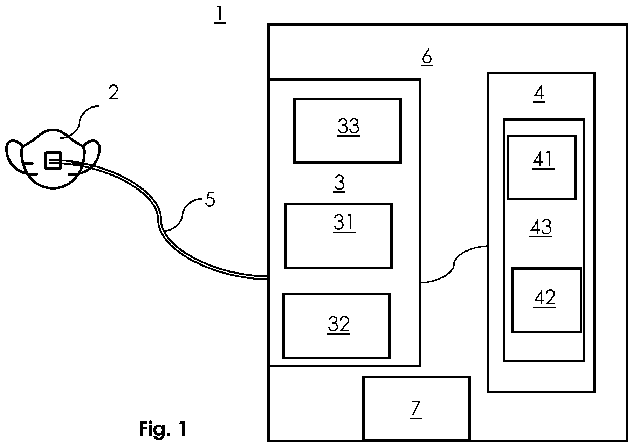

Fig. 1 shows an embodiment of a system 1 for respiratory oscillometry comprising a ventilation interface 2, an excitation means 31, a ventilation sensor 32, an excitation response processing means 41 and an excitation signal generation means 42. The system comprises preferably further an output means 7. Respiratory oscillometry is sometimes also called Forced Oscillation Technique.

The system can be a (stand-alone) respiratory oscillometry system for measuring the respiratory system of a human/patient. The respiratory oscillometry system is specially adapted for spontaneous breathing humans/patients, i.e. naturally breathing humans/patients which do not need any support in breathing. In such an embodiment, the system for respiratory oscillometry would not be connected to a mechanical ventilation system for assisting or replacing the breathing of the patient.

However, the system for respiratory oscillometry can also be integrated in a mechanical ventilation system. A mechanical ventilation system (short mechanical ventilator) assists or replaces the act of breathing in order to ventilate the patient. The mechanical ventilator delivers air (with or without added oxygen) into the patient's lung. It is commonly used in critical care settings when patients are unable to breath adequately on their own due to illness, injury, or anesthesia. In case of integration of the respiratory oscillometry system in a mechanical ventilator, the respiratory oscillometry system would further contain a ventilation unit 33 which generates and regulates airflow and/or pressure of the ventilation interface 2 and/or of the respiratory system of the patient. This ventilator unit could comprise filters, humidifiers, temperature adjusters for controlling the air entering the interface 2 and/or the patient. The ventilator unit 33 could contain then further sensors to monitor pressure, air flow and/or oxygen levels of the air provided to the interface 2 and/or to the patient. The ventilation sensor 32 can be realized by one of the sensors of the ventilation unit 33 or vice versa. The excitation means 31 could also be realized by a part of the ventilation unit 33. However, it would also be possible that the system for respiratory oscillometry is a stand-alone system (which could work without a mechanical ventilator), but could be connected to the mechanical ventilator, e.g. by connecting it to the conducts 5 of the ventilation of the patients. The mechanical ventilation unit 33 can operate on one or more of the following described ventilation modes. The ventilation modes are tailored to different patient needs. Volume-Controlled Ventilation (VCV) delivers a set tidal volume regardless of airway pressure, ensuring consistent ventilation. Pressure-Controlled Ventilation (PCV) delivers air until a preset pressure is reached, with tidal volume varying based on lung compliance. Assist-Control Ventilation (ACV) provides full support by delivering a set volume or pressure for each breath, triggered by the patient or the ventilator. Synchronized Intermittent Mandatory Ventilation (SIMV) combines mandatory breaths with spontaneous breathing, allowing the patient to breathe independently between set breaths. Pressure Support Ventilation (PSV) assists each spontaneous breath with a preset pressure, reducing effort while the patient controls breath timing. High-Frequency Oscillatory Ventilation (HFOV) uses very small tidal volumes at high frequencies (300-900 breaths per minute) to protect the lungs in severe conditions like ARDS. These modes provide varying levels of support based on the patient's condition and ability to breathe. The ventilation modes could also be grouped in mechanical ventilation which replaces the ventilation/breathing of the patient like VCV, HFOV or PCV (controlled ventilation) and in mechanical ventilation modes which just assist/support the patient in breathing like ACV, SIMV, PSV (assisted mechanical ventilation).

The ventilation interface 2 is configured to connect the system with the respiratory system of the patient. The ventilation interface 2 is preferably such that it provides an airtight connection between the respiratory system of the patient and the (air conduct 5 of the) system 1. The ventilation interface 2 could be a mouthpiece for connecting the (air conduct 5 of the) system 1 to the respiratory system of the patient via the mouth and/or nose. The mouthpiece could be realized as some mask covering mouth and/or nose. If only the mouth or nose is covered, the other one is closed. For example, if only the mouth is covered, the nose is normally closed with a nose clamp. For example, if only the nose would be covered (which is rare), then the mouth must be closed which could be achieved by the patient him/herself. The mouthpiece could also be something which is inserted in the mouth and sealed by the lips of the patient. The mouthpiece could for example be hold by the teeth of the patient in the correct position. Such mouthpieces as described are normally used for respiratory oscillometry systems for spontaneous breathing patient. For mechanical ventilation patients, the ventilation interface 2 could also be a ventilation tube inserted in the trachea, e.g. an endo-tracheal tube (inserted via the mouth) or a tracheostomy tube (inserted via a surgical opening directly in the trachea). However, any other ventilation interface 2 can be used.

The ventilation interface 2 is connected via air conducts 5 with the ventilation mechanics 3. The ventilation mechanics 3 contain the excitation means 31 and the ventilation sensor 32. The ventilation interface 2 and the ventilation mechanics 3 are normally arranged in distinct devices connected via an air tube 5 forming part of the air conduct 5. Air conduct 5 shall here refer to the complete conduct of air inside the ventilation mechanics up to the ventilation interface 2. The air tube 5 refer more to the connection between the air conducts in the ventilation interface 2 and the air conducts in the ventilation mechanics 3. However, the ventilation interface 2 could also be integrated in the ventilation mechanics 3 (or vice versa).

The excitation means 31 is configured to superimpose an oscillatory excitation on a ventilation of the patient based on an oscillatory excitation signal. Ventilation of the patient on which the oscillatory excitation is superimposed shall include the ventilation of natural or spontaneous breathing patients or the ventilation of patients caused by mechanical ventilation (assisted or replacing ventilation). The excitation means 31 is anything configured to generate the oscillatory excitation in the ventilation of the patient. The excitation means 31 can be a turbine, a fan, a vibrating membrane, a loudspeaker diaphragm or a piston connected with the air conduct 5 of the system 1. The excitation means 31 is preferably arranged in the ventilation mechanics 3 or respiratory oscillometer 6, however, it could also be integrated in the ventilation interface 2. The excitation signal will be described in more detail later. Since air conduct 5 is connected (in a sealed manner) with the respiratory system of the patient, the oscillatory excitation travels through the air in the air conduct 5 into the respiratory system of the patient. The oscillatory excitation are superimposed (added) on the tidal movement of the air in the respiratory system of the patient and the air conduct 5 due to the ventilation of the patient.

The ventilation sensor 32 is configured for measuring the ventilation of the patient. The measurement of the ventilation sensor 32 results in a ventilation measurement signal. The ventilation sensor comprises preferably a pressure sensor and/or a flow sensor. The pressure sensor measures the pressure in the ventilation mechanics 3, the air conduct 5, in the ventilation interface 2 and/or in the respiratory system of the patient. Preferably, the pressure sensor is arranged in the ventilation interface 2 or in the air tube 5 close to the ventilation interface 2 in order to get the pressure as close as possible to the respiratory system of the patient. The flow sensor measures the air flow in the air conduct 5, in the ventilation interface 2 and/or in the respiratory system of the patient. In other words, the flow sensor measures the air flow coming out or going in the respiratory system of the patient. The ventilation sensor 32 contains preferably both sensors. The ventilation measurement could be thus also a two-dimensional signal, a first dimension representing pressure over time and a second dimension representing the air flow over time. The ventilation sensor 2 is configured to perform a ventilation measurement (signal) while the excitation means 31 superimposes an oscillatory excitation over the ventilation of the patient. In this case, the ventilation measurement shall be called a ventilation excitation measurement.

The excitation response processing means 41 and excitation signal generation means 42 are preferably realized as software 43 running on a processor 4. The processor 4 is preferably arranged in a local device arranged close to the patient or ventilation interface 2. The processor 4 can be arranged in the same device as the ventilation mechanics 3. Preferably, the system 1 comprises a respiratory oscillometer (RO) device 6 which comprises the processor 4 and the ventilation mechanics 3 at least in part. The ventilation mechanics 3 arranged in the RO device 6 is connected via the air tube 5 with the ventilation interface 2. If the complete ventilation mechanics are arranged in the ventilation interface 2, there is no need to connect the RO device 6 with an air tube 5. If the RO device 6 contains the processor 4, but some or all of the ventilation mechanics 3 are arranged in the ventilation interface 2, then the (ventilation mechanics 3 included in the) ventilation interface 2 is electrically connected to the processor 4. This connection can be realized via a cable or via a wireless connection. Finally, it is also possible that the processor 4 is arranged in the ventilation interface 2 as well. For example, the ventilation mechanics and the processor 4 could be integrated fully in the ventilation interface 2 so that the system 1 is realized in one device. The processor 4 can obviously contain different sub-processors which are distributed over different devices of the system 1, e.g. one in the ventilation interface 2 and one in the RO device 6. It is further possible that the processor 4 or a sub-processor is arranged in a third device, e.g. a PC. It is also possible that the processor 4 or at least part of the functionalities of the processor 4 are realized by a remote server connected with the ventilation mechanics 3. Obviously, the functions of the processor have not to be realized as a software on a general purpose processor, but can fully or partly also be realized by corresponding electric circuitry instead of by software.

The excitation response processing means 41 is configured to determine based on the ventilation excitation measurement of the patient a response of the respiratory system on the oscillatory excitation signal and/or a parameter of the respiratory system of the patient (respiratory parameter). Preferably, the respiratory parameter is computed based on the response of the respiratory system. The excitation response processing means 41 computes preferably a frequency spectrum of the measured ventilation excitation measurement representing the oscillation components. The frequency spectrum represents normally the amplitude and/or phase of the oscillation components at each frequency. The frequency spectrum is preferably obtained by a Fourier transform of the ventilation excitation measurement or any other representation of the ventilation excitation measurement in the frequency domain. The Fourier transform shall include also other frequency spectra obtained from the Fourier transform like a power spectrum (square of the Fourier transform), amplitude of the Fourier transform, Real part of Fourier transform or Imaginary part of the Fourier transform, .... However, it would also be possible to represent the frequency spectrum in the time domain, e.g. by the autocorrelation function of the ventilation excitation measurement. In this case, the frequency of the autocorrelation function would correspond to the time delay and the square root of the autocorrelation function at a time delay to the amplitude of the Fourier transform at a corresponding frequency. In one embodiment, only the frequencies of the frequency spectrum which are also used for generating the excitation signal are used for determining the respiratory parameter. In another embodiment, also other frequencies of the frequency spectrum are used to obtain the respiratory parameter. It is also possible to obtain the respiratory parameter from the ventilation excitation measurement in the time domain, e.g. by fitting a model or function on the ventilation excitation measurement.

Before determining/computing the respiratory parameter from the frequency spectrum, the frequency spectrum is normally normalized. This is done for example based on a baseline measurement, i.e. a ventilation measurement from the ventilation sensor 32 from the same patient but without oscillatory excitation. In preferred embodiment, the ventilation reference measurement used also for generating the excitation signal can be used to obtain the baseline measurement. The frequency spectrum of the baseline measurement can be subtracted or divided or otherwise normalized from the frequency spectrum of the ventilation excitation measurement. Subsequently, the processing of the frequency spectrum of the ventilation measurement signal to obtain a feedback is explained for an example. The frequency spectrum used for the feedback calculation can be normalized or non-normalized without each time mentioning this.

The respiratory parameter can comprise simply the value of the frequency spectrum at a specific or more specific frequencies, normally the excited frequencies or a valued computed from this (this is normally called a non-parametric respiratory parameter). Such non-parametric parameters could be the impedance, the reactance and/or the resistance. Subsequently, an exemplary calculation for obtaining those parameters for a certain frequency are presented, when the ventilation excitation measurement contains pressure and flow rate of the ventilation.

The ventilation excitation measurement is subjected to Fourier Transform to decompose it into its frequency components. This yields complex frequency-domain representations of pressure P(f) and flow V'(f) at each oscillation frequency f. From these representations, the impedance Z(f) of the respiratory system is determined by calculating the ratio of the pressure signal to the flow signal at each frequency, expressed mathematically as: $Z \left(f\right) = \frac{P \left(f\right)}{V ′ \left(f\right)}$ Impedance Z(f) is a complex quantity that comprises two components: resistance R(f) and reactance X(f). The resistance R(f) is derived as the real part of the impedance and reflects the opposition to airflow due to frictional forces in the airways. The reactance X(f) is derived as the imaginary part of the impedance and represents the combined effects of airway inertance and lung tissue compliance. These relationships can be expressed as: $R \left(f\right) = Re \left(Z\left(f\right)\right)$ and $X \left(f\right) = Im \left(Z\left(f\right)\right)$ The magnitude or amplitude of the impedance IZ(f)I is calculated as the square root of the sum of the squares of the resistance and reactance: $\left|Z\left(f\right)\right| = \sqrt{R {\left(f\right)}^{2} + X {\left(f\right)}^{2}}$ The analysis of these parameters provides insight into the mechanical properties of the respiratory system, such as airway resistance, lung tissue compliance, and the inertial properties of the airways. This method enables the identification and characterization of conditions affecting the airways and lung function, including obstructive and restrictive pulmonary diseases. These values allow an assessment of the respiratory system (airways and or lungs). High resistance values typically indicate airway obstruction, such as in conditions like asthma or COPD. Reactance reflects the elastic and inertial properties of the lungs; negative reactance suggests restrictive lung diseases, such as pulmonary fibrosis or stiff lungs, while highly negative reactance at low frequencies may point to small airway disease. Impedance represents the overall opposition to airflow in the respiratory system, combining both resistance and reactance into a single measure of lung function. On the other side, excitation frequencies and the corresponding feedback at those excitation frequencies will have different meanings. Higher frequencies assess large airways and lower frequencies assess small airways and lung tissue.

Other respiratory parameters can be obtained by special models (parametric respiratory parameter). Such models can be fitted on the ventilation excitation measurement or on its frequency spectrum. For example, a single compartment model can be fitted on the ventilation excitation measurement. Such a parameter could be the compliance to evaluate the stiffness of the lungs or the resistance for evaluating obstructions of the upper airways.

It is obvious that also other ventilation excitation measurement parameters than pressure and flow rate can be analysed, e.g. only one of them or different parameters. It is also clear that other feedback parameters than the described can be used or even as explained above non-parametric feedback can be used.

The excitation signal generation means 42 is configured to generate the oscillatory excitation signal based on a patient specific data. In a preferred embodiment, the patient specific data contains preferably a ventilation reference measurement of the ventilation of the patient. The ventilation reference measurement is preferably a measurement of the ventilation of the patient without the oscillation excitation. The ventilation reference measurement contains preferably the same parameter as the ventilation excitation measurement. Preferably, the ventilation reference measurement is measured with the ventilation sensor 32. Using the same sensor 32 for ventilation reference measurement and for the ventilation excitation measurement assures that the sensor specific noise is considered for the ventilation excitation signal. If the RO is integrated in a mechanical ventilation, the ventilation measurement of the mechanical ventilator can be used as ventilation reference measurement. However, it would also be possible to use a ventilation reference measurement realized with a different sensor. It would also be possible that the ventilation reference measurement is rather a predicted model of the respiratory system of the patient, than directly a measurement of the patient. The signal design could be improved iteratively using simulation results, avoiding the need for a separate ventilation measurement. This could be especially interesting for outpatient measurements. As mentioned above, the ventilation reference measurement could be used both as baseline measurement and as ventilation reference measurement. However, it would be also possible to do a distinct baseline measurement and a ventilation reference measurement or to do not use a baseline measurement for normalization of the ventilation excitation measurement. In another embodiment, the ventilation reference measurement could also be ventilation measurement performed during an oscillation excitation. For example, a previous ventilation excitation measurement of the patient can be used as ventilation reference measurement. In this case, the same procedure as below would apply, but optionally the previous ventilation excitation measurement could be corrected by the previous oscillatory excitation signal used for the superimposed ventilation excitation on the previous ventilation excitation measurement to obtain the ventilation reference measurement. This could be useful for continuous monitoring applications, e.g. during mechanical ventilation of a patient.

The patient specific data can contain further other patient specific data like one or more of the disease type of the patient, the disease state of the patient, general patient specific data (sex, lung volume, chest size, ...), previous measurements. Previous measurements can include previous feedback value obtained before. This could help to increase maybe the amplitude for feedback values which are critical to verify, if they are correct. Previous measurements could also include previous ventilation reference measurements (preferably by the ventilation sensor 32 and/or being different from ventilation reference measurement above) and/or previous ventilation excitation measurements. This could help to further improve the oscillatory excitation signal for the patient.

Even if it is preferred that the patient specific data contain the ventilation reference measurement, it would however also be possible to generate the excitation signal based on a patient group in which the patient is classified based on its other patient specific data like based on its disease type, its disease state and/or its other patient specific data. However, in this case, preferably the number of frequencies, frequency values, amplitudes and/or phases of the excitation frequencies are determined based on a representative ventilation reference measurement of one or more patients of this patient group.

The excitation signal generated based on the invention contains preferably (non-zero) oscillation components at frequencies lower than 5 Hertz (Hz). RO at frequencies below 5 Hz allow to analyse the peripheral region of the lungs. Each (non-zero) oscillation component of the oscillatory excitation signal at a frequency is subsequently abbreviated an excitation frequency. Most state-of-the-art RO use only frequencies above 5Hz because the frequencies below 5 Hz are highly disturbed by the breathing activity of the patient and/or the mechanical ventilation mode which act in the range of 1-3 Hz. Also, patient activity can generate significant noise in these frequencies. However, the current patient specific excitation signal generation allows to use excitation frequencies below 5 Hz. Obviously, the method according to the invention can also be used for excitation frequencies at 5Hz and above.

The oscillatory excitation signal (short also excitation signal) is composed of at least one excitation frequency, preferably a plurality of excitation frequencies. According to the invention, the number of the excitation frequencies, the amplitude of each excitation frequency and/or the phase of each excitation frequency are selected/chosen based on the patient specific data, preferably based on the ventilation reference measurement. Preferably, also the frequency values of the excitation frequency can be chosen based on the patient specific data, preferably based on the ventilation reference measurement. The number of the excitation frequencies, the frequency value of each excitation frequency, the amplitude of each excitation frequency and the phase of each excitation frequency are signal parameters of the ventilation excitation signal.

The oscillatory excitation signal can be generated for example by a formula based on a plurality of K excitation waves k=1, ..., K, each described by its excitation frequency fₑ(k), its excitation amplitude Aₑ(k) and its excitation phase Φₖ. The excitation waves can be described by a cosine function, a sine function, a combination of cosine and sine function or an exponential function. In case of describing the excitation waves with a cosine function, the oscillatory excitation pₑ(t) signal over time t could be described as ${p}_{e} \left(t\right) = {\sum }_{k = 1}^{K} {A}_{e} \left(k\right) cos \left(2{πf}_{e}\left(k\right)t+{ϕ}_{k}\right) ,$ The oscillatory excitation signal is preferably sampled equidistantly using t=(0, 1, ..., N-1)Tₛ, where N is the total number of data points and Tₛ is the sampling period. The frequency resolution fᵣₑₛ is preferably defined by the total measurement time T of the ventilation reference measurement as : ${f}_{res} = \frac{1}{T} ,$ with $T = {NT}_{s} .$

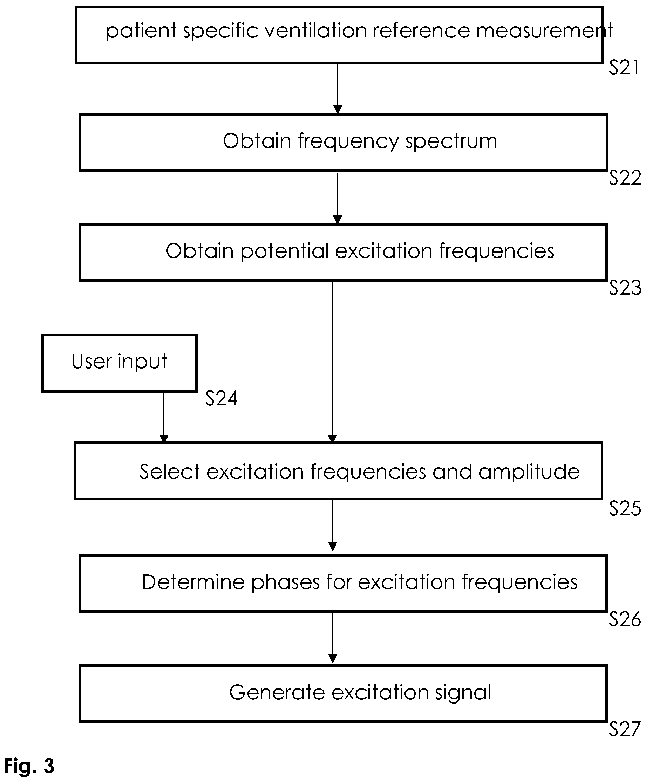

An exemplary generation of the oscillation excitation signal will be described in more detail below with Fig. 3.

The output means 7 is configured to output the determined respiratory parameter. The output means is preferably a visual or audio output means to output the respiratory parameter visually or auditive. The visual output means is preferably a display. The audio output means is preferably a loudspeaker. The display is arranged preferably in the respiratory oscillometer 6. However, the output means 7 could also be a communication interface to send the respiratory data to a user device to read out the respiratory parameter and/or display it. The communication interface can be a socket for receiving a cable connection, e.g. an USB socket. The communication interface could also be a wireless communication interface, e.g. a Bluetooth (registered trademark) interface, a WLAN interface, a cellular phone network interface like GPRS, 3G, 4G, 5G, 6G, an IOT interface like LoRa, or other connection types.

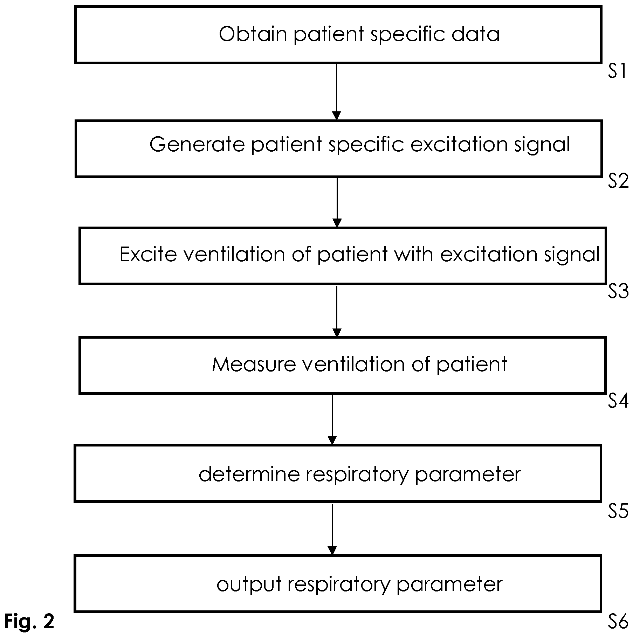

Fig. 2 shows an exemplary method for oscillatory respiratory.

In step S1, patient specific data are obtained. The patient specific data contain preferably the ventilation reference measurement of the patient (as explained in more detail above). Additionally or alternatively, the patient specific data could also comprise data from previous measurements. Such data could comprise the oscillation excitation signal of a previous measurement, the ventilation reference measurement of the previous measurement and/or the ventilation excitation measurement of the previous measurement and/or the feedback obtained from the previous measurement.

In step S2, the patient specific oscillation excitation signal is generated based on the patient specific data, preferably based on the ventilation reference measurement. This step is partly already described above for the functionality of the excitation signal generation means 31 and will be described below in more detail with Fig. 3.

In step S3, the ventilation of the patient is superposed an oscillation excitation based on the generated oscillation excitation signal generated in step S2.

In step S4, the ventilation of the patient is measured with the ventilation sensor 32 during the oscillation excitation of the ventilation of the patient in order to obtain the ventilation excitation measurement. Thus, steps S3 and S4 must be performed at the same time. This step is described in more detail with the functionality of the ventilation sensor 32.

In step S5, an excitation response or the respiratory parameter is computed based on the ventilation excitation measurement obtained in step S4. This step is described in more detail with the functionality of the excitation response processing means 41.

In step S6, the respiratory parameter computed/determined in step S5 is given out through the output means 7.

A computer program according to the invention could be configured, when executed on a processor, to perform just the steps of S1 and S2 and give out to a respiratory oscillator the generated excitation signal. In this case, the computer program could be used to upgrade existing ROs by the functionality to design the excitation signal patient specific. The computer program could however also be configured to control the respiratory oscillator and perform also steps S3, controlling the excitation means 31 to excite the ventilation of the patient with the generated oscillatory excitation signal of S2, and step S4, receiving the ventilation excitation measurement from the ventilation sensor 32, and S5, and S6, giving out the respiratory parameter to the output means 7. In case of an integration of the RO into a mechanical ventilator, the computer program could be further configured to control the mechanical ventilator.

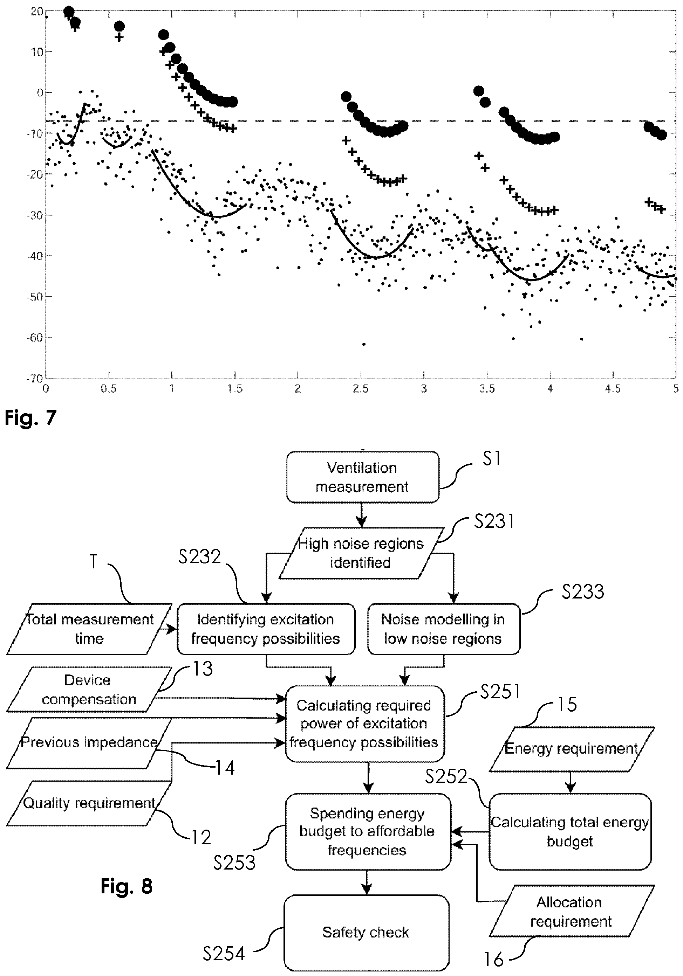

Fig. 3 shows now an embodiment for generating the oscillation excitation signal. Fig. 8 shows a more detailed schematic for another example how to find the excitation frequencies and their amplitudes. Even though Fig. 8 is more detailed as Fig. 3, it shall not limit the invention to all the shown steps.

In step S21, the ventilation reference measurement is provided. In the subsequent example, the pressure measurement of the ventilation of the patient is used.

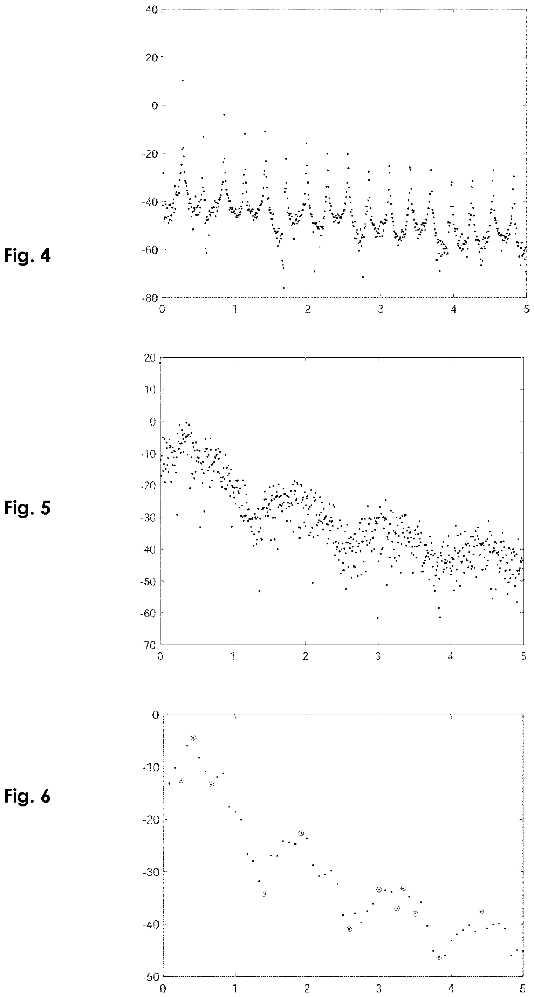

In step S22, a frequency spectrum of the ventilation reference measurement is computed. The frequency spectrum is preferably a complex frequency spectrum, i.e. a frequency spectrum whose spectrum values are complex numbers with a real part and an imaginary part. The frequency spectrum is preferably a Fourier transform of the ventilation reference measurement (including also a Fourier transform of a processed ventilation reference measurement). The frequency spectrum can be a complex Fourier transform, the amplitude of the Fourier transform, the square of the amplitude of the Fourier transform (also called power spectrum), the phase of the Fourier transform, the real part or the imaginary part of the Fourier transform. The frequency spectrum can also be computed based on a different frequency domain representation than the Fourier transform. The frequency spectrum could even be represented in the time domain by the autocorrelation function of the ventilation reference measurement which would be an equivalent representation mathematically to the power spectrum. In Fig. 4, the frequency spectrum of ventilation of a volume controlled mechanically ventilated patient is shown, computed by the following equation for the discrete Fourier transformation: ${P}_{VEN} \left(r\right) = {\left|\frac{1}{\sqrt{N}}{\sum }_{n = 0}^{N - 1} {p}_{d} \left(n\right) {e}^{- \frac{j 2 πrn}{N}}\right|}^{2}$ where p_{d} is the sampled measured pressure signal at the airway opening, N the total number of datapoints and r = (0,1 ,...,N-1) the Fourier transform bin. In Fig. 5, the frequency spectrum of a ventilation of a pressure supported mechanically ventilated patient is shown as an example of an assisted mechanical ventilation. As can be well seen, the volume-controlled ventilation shows clear harmonics which make it more easy to select the frequency values of excitation frequencies. Due the natural breathing of the patient in pressure-supported ventilation, these clear harmonics disappear in the noise and the design of the excitation signal is much more difficult. In Fig. 4 to 7 the amplitude of the Fourier transform of a pressure signal in decibel Hectopascal (dBhPa) is shown for the frequency values between 0 and 5 Hz. The ventilation reference measurement underlying the frequency spectrum in Fig. 4 and 5 was here T=120 seconds long plus 6 seconds for transient removal. It is clear that other representations of the frequency spectrum and other measurements than the pressure can be used for designing the excitation signal. The example spectrum of Fig. 5 will be used to illustrate the design of the excitation signal without any limitation of the invention.

In step S23, potential excitation frequencies are determined based on the frequency spectrum, preferably based on the amplitude of the frequency spectrum or based on the power spectrum.

Preferably, the frequency spectrum is used to determine high and/or low noise regions (see step S231) or low noise frequency values/regions which can be used as potential excitation frequencies. Preferably, the low noise regions are identified based on local minima and/or maxima. Those can be found for example, by identifying minima and maxima in a smoothed frequency spectrum as shown for example in Fig. 6 and defined by : ${M}_{Minima} = \left\{r\left|{S}_{VEN}\left(r\right) is local min\right.\right\} ,$ ${M}_{Maxima} = \left\{r\left|{S}_{VEN}\left(r\right) is local max\right.\right\} ,$ ${M}_{extrema} = {M}_{Minima} ∪ {M}_{Maxima}$ Where S_{VEN}(r) is a smoothed version of P_{VEN}(r). In Fig. 6, a median filter providing a median of five neighbouring samples and a subsequent average filter providing an average of two neighbouring samples (of the signal to which the median filter was applied) is applied to the frequency spectrum of Fig. 5, of P_{VEN}(r). The maxima and minima are highlighted by a circle around the respective sample points of the smoothed frequency spectrum. The potential frequency values for potential excitation frequencies can then be obtained by comparing the frequency spectrum value (preferably the power spectrum or amplitude) with a threshold computed based on the neighbouring minimum and maximum. The threshold can be for example half of the sum of the frequency spectrum value at the next local minimum and the next local maximum. The threshold could be computed by the following equation: ${S}_{threshold} \left(m\right) = \left(\frac{{S}_{VEN} \left({M}_{extrema}\left(m\right)\right) + {S}_{VEN} \left({M}_{extrema}\left(m+1\right)\right)}{2}\right)$ With m=(1, 2, ..., M-1), where M is the number of the local extrema in Mₑₓₜᵣₑₘₐ. Thus, the threshold is dynamic and will vary depending on the respective frequency region. All frequency spectrum values being below the threshold can be considered as potential frequency values for excitation frequencies. $fe " = fe ′ \ fven$ with f_{e'}, the set of all excitation frequencies defined as: ${f}_{e ′} = \left(2r+1\right) 3 {f}_{res} with {f}_{res} = \frac{1}{T}$ and the frequencies-to-avoid, fᵥₑₙ array: ${f}_{ven} = \left\{\left.r\right|{M}_{extrema}\left(m\right)\leq r\leq {M}_{extrema}\left(m+1\right),{S}_{VEN}\left(r\right)>{S}_{threshold}\left(m\right)\right\} .$ This allows to determine low noise frequency regions in different frequency regions and to identify possible excitation frequencies. This would correspond to the steps S231 and S232 in Fig. 8. When working with discrete frequencies, the potential excitation frequencies are preferably a subset of the frequencies f_{e'} resulting from the frequencies bins r used in the Fourier transform defined by the duration T of the ventilation reference measurement (and being below the above mentioned threshold). So, preferably the same excitation frequencies are used as resulting from the discrete Fourier transformation bins. However, in an alternative embodiment, it would also be possible to use different excitation frequencies, e.g. obtained by interpolation of the values of the Fourier transform bins. This is obviously only one method to determine low noise frequency regions in the frequency spectrum as potential frequency values/regions for excitation frequencies of the excitation signal. For example, the minimal noise regions can be modelled by fitting noise models on the spectrum. In Fig. 7, the continuous lines show the fitted noise models in the low noise regions. The described concepts apply obviously only when the frequency spectrum contains local minima and maxima. Otherwise, fixed frequency regions can be used. Wherein in each frequency region, the frequency value with the lowest amplitude is selected as potential excitation frequency.

In step S25, the amplitude and the frequency values of the excitation frequencies are selected and preferably also the number of excitation frequencies. Preferably, a required amplitude is calculated for each (potential) excitation frequency (see step S251). The (required) amplitude of the excitation frequencies or of the potential excitation frequencies are computed based on the (smoothed or otherwise processed) frequency spectrum, and preferably by an additional amplitude value.
The smoothed or otherwise processed frequency spectrum is preferably the estimated noise function Gᵢ(ω(k")) of the frequency spectrum, , i = (1,2,...,l) where l is the total number of minima in M_{Minima} (see step S233). Preferably, a local noise model is fitted to the frequency spectrum in each low frequency region determined above. The function Gᵢ is a second-degree polynomial fit to P_{VEN,i}(r), representing the local noise power of all frequency bins within the region containing the excitation frequencies of subset f_{e"-Minima,i}, which relates to f_{e"} according to ${f}_{e "} \left(k"\right) = {∪}_{i = 1}^{I} {f}_{e " - Minima , i} \left(k"\right)$ To improve polynomial fitting, the regions are extended by 10 samples on each side. However, it would also be possible to obtain the noise function differently, e.g. by fitting a global noise model or simply by smoothing/filtering the frequency spectrum.

The additional value can depend preferably on one or more of the quality requirement 12, the device compensation 13 and the previous impedance 14. These parameters can be user inputs S24 or could be obtained from a database like the previous impedance 13 from the last measurement. If the additional value depends on two or three of the device compensation 14, the previous impedance 13 and the quality requirement 12, the additional value can be decomposed in two or three of a device compensation component, an impedance component and a quality requirement component, respectively. The additional amplitude value (or its components) can be an absolute additional amplitude value added on top of the amplitude of the frequency spectrum at the (potential) excitation frequency or can be a frequency or amplitude dependant additional amplitude value increasing the amplitude of the smoothed or processed frequency spectrum. The frequency or amplitude dependent additional amplitude value could be simply a relative additional amplitude value increasing the amplitude relative to the amplitude of the frequency spectrum at the (potential) excitation frequency by a fix percentage value or in other words increase the amplitude linear with respect to the amplitude value, e.g. an increase by 10%. It is however also possible to use more complex functions of the frequency or amplitude dependent additional amplitude value.

The additional value or the quality requirement component is preferably obtained based on a quality requirement 12 obtained from a user input S24 or from a predetermined configuration. The quality requirement 12 could be a signal to noise ratio SNR_{Req}. The signal to noise ratio could be selected depending on the respiratory parameter to be determined in step S5. For example, for a non-parametric respiratory parameter like an impedance at a certain frequency, a higher signal to noise ratio could be selected than for a respiratory parameter obtained by a parametric model. For example, the SNR for a non-parametric respiratory parameter could be 20dB and the SNR for a parametric respiratory parameter could be 6dB. The user could maybe select via user input the desired respiratory parameter which automatically sets the higher or lower required signal to noise ratio. The user could also set the SNR or other quality requirement directly as a user input in step S24. However, it is also possible that the SNR or other quality requirement 12 is predetermined. The quality requirement component and/or the additional value could be calculated based on a formulate depending on the SNR required and the (smoothed and/or processed) frequency spectrum, e.g. based on the following formula ${A}_{T} \left({f}_{e "}\left(k"\right)\right) = 2 ⋅ {10}^{\left(\frac{{SNR}_{Req}}{10}\right)} {G}_{i} \left(2{πf}_{e "}\left(k"\right)\right) .$ With A_{T}(f_{e''}(k'')) the target amplitudes or the quality requirement component, SNR_{Req} the required signal to noise ratio as quality requirement, Gᵢ(ω(k")) the estimated local noise power function and k" = (1,2,...,K") where K" is the number of available excitation frequencies.

The device compensation component could compensate for a frequency dependent device specific compensation function considering that the ventilator or the ventilation excitation means 31 does not work equally for all (available or potential) excitation frequencies. The compensation component could take account for the ventilator performance constraints. Performance degradation is particularly present at higher frequencies and elevated flow rates. For example, ventilators optimized for operation in a certain frequency range, e.g. 0.1 to 0.4 Hz, exhibit increasing amplitude attenuation beyond this range due to turbine inertia and RPM limitations. Since the flow rate depends on the load attached, a load-dependent compensation model could be applied, correcting for frequency-specific amplitude losses. The device compensation component A_{comp}(fₑ(k)) can be determined for example based on the pressure loss compared to a target pressure of a ventilator (in case the RO is included in a ventilator).

The additional amplitude function can for example be defined by a combination (e.g. the sum) of the quality requirement component and the device compensation component ${A}_{e} \left(k\right) = {A}_{T} \left({f}_{e}\left(k\right)\right) + {A}_{Comp} \left({f}_{e}\left(k\right)\right) ,$ Such a frequency or amplitude dependent additional amplitude could increase the (relative) additional amplitude value versus higher frequencies as shown in Fig. 7. The crosses represent the possible excitation frequency-amplitudes combinations which have an amplitude that is 20dB higher than the modelled noise (continuous line). The large black dots represents the same excitation frequency possibilities but where the amplitude of the crosses is supplemented with the additional relative amplitude determined as a frequency dependent function. In this case a relative increase of the additional amplitude over the frequencies is noticeable. The frequency or amplitude dependent additional amplitude function is preferably determined based on the system or based on the ventilator. The user can for example select in S24 the type of additional amplitude value for obtaining the amplitude of the (potential) excitation frequencies or the parameters to calculate the additional amplitude value like the device compensation 13 and/or the quality requirement 12.

In order to select the frequency values and/or the number of excitation frequencies, preferably a total available amplitude budget is introduced (see step S252). The total amplitude value of the excitation signal must be smaller than the total available amplitude budget. The total amplitude value of the excitation signal can be defined in many ways, like a value proportional to or depending on the sum of the absolute values of all the amplitudes of the excitation frequencies or like a value proportional or depending on to the sum of all the squared values of the amplitudes of the excitation frequencies (like in the example described below). However, other definitions of the total amplitude value are possible as well. Subsequently, the total amplitude value of the excitation signal could be defined as a total energy being T times the RMS²ₜₒₜₐₗ with ${RMS}_{total}^{2} = {\sum }_{k = 1}^{K} \frac{{A}_{e} {\left(k\right)}^{2}}{2} ,$
where K represents the number of excitation waves k (or excitation frequencies) and Aₑ(k) the amplitude of each excitation wave k. The total amplitude value could also be defined as a total power like the RMS²ₜₒₜₐₗ.
The available amplitude budget can now be defined based on the definition of the total amplitude value of the excitation signal and based on clinical constraints like for example the peak-to-peak limitation for the excitation signal. The peak-to-peak limitation, which relates to RMS via the crest factor (Cr): $Cr = \frac{{p}_{peak}}{{p}_{{RMS}_{total}}} ,$
where pₚₑₐₖ denotes the peak value of the signal, and p_{RMS}, ₜₒₜₐₗ is the RMS of the pressure signal (i.e. the square root of the above formula). Considering the desired clinical peak-to-peak limitation, simulations showed that a Crest factor should not be larger than Cr = 2.3. Thus, considering further clinical requirements of the maximum peak values pₚₑₐₖ, the total available amplitude budget is determined by an upper RMS bound of P_{RMS,total} ≈0.065 kPa , ensuring compliance with the ERS-imposed 0.3 kPa peak-to-peak limit. These values refer however only to one example of clinical constraints and are not limiting the invention. It can be said that preferably the total available amplitude budget should relate to an upper boundary of the squared power or the RMS to be below 0,1 kPa. Even if this is a preferred way to define the available total amplitude budget, it could be defined by different methods. As already mentioned, this total available amplitude budget can be set by energy requirement 15 input by a user input S24 by the clinical users so that for very sensitive patients or patients at risk, the total available amplitude budget could be reduced to reduce the risk of the excitation signal for the ventilation of the patient.

In Fig. 7, the horizontal dashed line shows an exemplary total amplitude budget. In case of the black thick dots used as amplitudes for the potential excitation frequencies, only the potential excitation frequencies above 2,4 Hz (in frequency regions 2,4-2,7 Hz, 3,7-4 Hz and 4,8-5Hz) would fall under the total available amplitude budget. If, the excitation frequency in the first region at about 2,5 Hz is selected, the complete amplitude budget is used and would not allow to select a second excitation frequency. Thus, the selection of two excitation frequencies at about 3,9 Hz and at 5 Hz might still fall under the amplitude budget, but would have the disadvantage to miss lower frequency excitation. Depending on the priority of the user, the optimization strategy could be selected in step S24 by a user input. The optimization strategy could be also called the allocation requirement 16 as it defines the strategy how the available total budget is allocated to different excitation frequencies. In one strategy, the excitation frequencies with the lowest costs, i.e. with the lowest required amplitude are selected to obtain a maximum number of excitation frequencies. This strategy could be optimized by constraints to have a minimum distance between neighbouring excitation frequencies in order to have not all excitation frequencies in the same frequency range. In another optimization strategy, the selected excitation frequencies are selected to have as much excitation frequencies as possible with the excitation frequencies distributed optimally over a defined frequency range, preferably below 5 Hz, preferably between 1 and 5 Hz. In another optimization strategy, the excitation frequencies with the lowest cost or required amplitude in defined frequency regions are selected. The defined frequency regions could be pre-defined or computed based on the frequency spectrum (e.g. each low noise region corresponds to one frequency region). The optimization strategy or the allocation requirement 16 could be pre-defined or could be variable based on a user input S24. For example, one frequency value could be selected in each low noise region or one in each predetermined frequency region. Another optimization strategy would be to choose the frequency value with the lowest possible frequency value for the excitation frequency. The above strategies could obviously also combined. On the other hand, with the crosses as amplitude values of the potential excitation frequencies, it would also be possible to select an excitation frequency at 1,5 Hz or maybe three excitation frequencies at 2,6 Hz, 3,9 Hz and 5 Hz. Thus, the total amplitude budget in combination with the frequency spectrum of the ventilation reference measurement allows to select the number of excitation frequencies, their amplitude and their frequency values specific for a patient. Like this, the excitation signal can be designed such that RO measurements will be possible even in the high noise regions below 5Hz without disturbing or damaging the patient due to the controlled total amplitude budget.

Since the lowest noise is at the highest frequency, an optimization which selects the excitation frequencies with the lowest necessary amplitude would very often lead to most of the excitation frequencies arranged at the highest frequency values. Therefore, it is interesting to divide the spectrum into a plurality of frequency regions and select only one excitation frequency in each frequency region until the total amplitude budget is spent on the selected excitation frequencies. The frequency regions can be predetermined, e.g. 0-1 Hz, 1-2 Hz, 2-3 Hz, 3-4 Hz, 4-5 Hz. The frequency regions can be also selected based on the low noise regions determined in step S23, e.g. 0,8-1,6 Hz, 2,3-2,8 Hz, 3,5-4,2 Hz and 4,6-5Hz. Obviously, these low noise regions could be different for a different measurement. The frequency regions could also be defined in a fixed way. Preferably at least three different frequency region, even more preferably at least four different frequency regions, preferably in the frequency range below 5 Hz are selected. Depending on the selected order in which frequency regions are treated, the optimization objective can be chosen. When the frequency regions from the lowest frequency to the highest frequency are treated, low frequencies are favoured and probably the number of excitation frequencies is reduced. For example, for the low noise frequency regions in Fig. 7 with the black filled circles as potential amplitudes, there would be no excitation frequency selected in the first frequency region between 0,8 and 1,6 Hz, because their amplitudes are all over the total amplitude budget. In the second frequency region, there would probably be selected the frequency of 2,6 Hz, but would not leave any more any amplitude budget for the remaining frequency regions. Starting from the highest frequency region on the other side could lead to many excitation frequencies in the highest frequency region and missing ones in the lower frequency region. A compromise could be to jump from at least two of frequency regions at high frequencies, at low frequencies and at frequencies in the centre (e.g. at around 2,5 Hz). For example, when starting start with one of the highest frequency region at 4,6 Hz - 5 Hz, it would probably select a frequency value of 5 Hz (considering this time the crosses as amplitudes) and then jump to the lowest frequency region from 0,8-1,6 Hz which might have at 1,5 Hz some excitation frequencies under the total budget line, but with the amplitude spent for the excitation frequency at 5 Hz, there is probably not enough amplitude budget left for this excitation frequency in the lowest frequency region and as a consequence, the procedure would go to the next higher frequency region 2,3-2,8 Hz where there would be sufficient amplitude budget to add the excitation frequency at 2,6 Hz. The values are only exemplary and not limiting the disclosure.

The total amplitude budget is preferably variable by a user input, for example, if a user experience disturbances by the oscillatory excitations, then the total amplitude budget can be reduced. Also, if the oscillatory excitation leads to problems of the ventilation of the patient, the total amplitude budget must be reduced. The total amplitude budget is normally also selected based on the type of patient. For example, for a healthy patient the total amplitude budget can be selected higher than for a vulnerable patient during assisted mechanical ventilation.

The selection of the excitation frequencies could depend also on previous impedance 14 obtained from previous excitation measurements. The frequency regions with the highest curvature of the impedance could be identified and used as prioritized frequency regions in step S253. Alternatively or in addition, is also possible to use these identified frequency regions to increase the required amplitude in step S251 in these identified frequency regions in order to have a better measurement for those frequency regions.

In step S26, the phases of the excitation frequencies are determined. The phases of the excitation frequencies selected in step S25 can be selected based on the phases of the frequency spectrum at the selected excitation frequencies. One solution would be to calculate multiple sets of random phases for the excitation frequencies and select the set which provides the lowest peak values of the excitation signal. Alternatively or in addition, the phases can be determined also to correspond to the phases of the other excitation frequencies or by a clipping algorithm or by a crest factor minimization algorithm.

In step S27, with the phase and amplitude for each excitation frequency, the excitation signal in the time domain could be generated and superimposed on the ventilation of the patient or scaled before the application to the patient. Given the upper bound used for the crest factor, the designed excitation signal can be scaled to maximize its energy while maintaining compliance with peak-to-peak constraints. ${p}_{final} \left(t\right) = {p}_{e} \left(t\right) \frac{{p}_{Limit}}{{p}_{amplitude}}$ With p_{Limit} the RMS or peak limit value for the designed excitation signal pₑ(t) where p_{amplitude} is the corresponding RMS or peak value before the scaling. The peak-to-peak limit is verified prior to apply the signal (see step S254).

It should be understood that the present invention is not limited to the described embodiments and that variations can be applied without going outside of the scope of the claims.

## Claims

1. System for respiratory oscillometry for measuring a respiratory parameter of a respiratory system of a patient comprising: a ventilation interface (2) to connect the system with the respiratory system of the patient; an excitation means (31) configured to superimpose an oscillatory excitation on a ventilation of the patient based on an oscillatory excitation signal; a ventilation sensor (32) for measuring the ventilation of the patient, wherein the ventilation sensor is configured to perform a ventilation excitation measurement of the ventilation of the patient superimposed by the oscillatory excitation; an excitation response processing means (41) configured to determine based on the ventilation excitation measurement of the patient a response of the respiratory system on the oscillatory excitation signal, and to determine based on the response determined the respiratory parameter of the respiratory system; an output means (7) for outputting the determined respiratory parameter of the respiratory system; an excitation signal generation means (42) configured to generate the oscillatory excitation signal with one or more excitation frequencies, wherein one or more signal parameters of the oscillatory excitation signal are determined based on a ventilation reference measurement of the patient, **characterized in that** the one or more signal parameters determined are one or more of the following: number of the one or more excitation frequencies, an amplitude of the one or more excitation frequencies and a phase of the one or more excitation frequencies.

2. System according to claim 1, wherein the ventilation reference measurement is a measurement of the ventilation of the patient without superimposed excitation oscillation.

3. System according to claim 2, wherein the ventilation reference measurement is a measurement of the ventilation of the patient with an oscillatory excitation superimposed on the ventilation of the patient.

4. System according to one of claims 1 to 3, wherein the ventilation reference measurement is measured by the ventilation sensor.

5. System according to one of claims 1 to 4, wherein a frequency spectrum of the ventilation reference measurement is computed, wherein the amplitude of each of the one or more excitation frequencies are determined based on the frequency spectrum of the ventilation references measurement.

6. System according to claim 5, wherein the amplitude for each excitation frequency depends on the frequency spectrum at a frequency value of this excitation frequency and a predetermined additional amplitude value.

7. System according to claim 6, wherein the additional amplitude value depends on a user input or depends on a measurement objective which can be selected by a user input.

8. System according to one of claims 5 to 7, wherein the one or more signal parameters are determined further based on a total amplitude budget.

9. System according to claim 8, wherein the total amplitude budget depends on user input.

10. System according to claim 8 or 9, wherein the number of the excitation frequencies are determined based on the determined amplitude of each excitation frequency and the total amplitude budget available.

11. System according to claim 10, wherein the number of excitation frequencies, the frequency values of the excitation frequencies and the amplitudes of the excitation frequencies are selected by an optimization criterium distributing the total amplitude budget over the determined number of excitation frequencies.

12. System according to claim 11, wherein the frequency values of the frequency spectrum is divided into a plurality of predefined frequency regions, wherein for each predefined frequency region at least one excitation frequency is selected based on a lowest necessary amplitude in this frequency region, if a sufficient amplitude budget is left to not exceed the total amplitude budget.

13. System according to one of claims 5 to 12, wherein the frequency values and their respective amplitudes of each of the one or more excitation frequencies are determined based on the frequency spectrum.

14. System according to one of the previous claims, wherein the excitation signal comprises excitation frequencies smaller than 4 Hz.

15. Mechanical ventilator comprising a system for respiratory oscillometry according to one of the previous claims.
